# EUROPEAN PATENT SPECIFICATION

(11) **EP 3 407 921 B1**
(45) Date of publication and mention of the grant of the patent: **30.09.2026**
(21) Application number: 17705302.2
(22) Date of filing: 26.01.2017
(51) Int. Cl.: A61L 2/07, A61L 2/26, B01L 1/02

(54) **PROCESS FOR THE TRANSPORT OF A STERILE PARTICULATE BULK MATERIAL AND ITS TRANSFER INTO AN ISOLATOR, AS WELL AS A CONTAINER, AN ISOLATOR AND A COMBINATION OF AN ISOLATOR AND A CONTAINER**
VERFAHREN ZUM TRANSPORT EINES STERILEN SCHÜTTGUTMATERIALS UND DESSEN TRANSFER IN EINE ISOLIERKAMMER SOWIE EIN BEHÄLTER, EINE ISOLIERKAMMER UND EINE KOMBINATION AUS EINER ISOLIERKAMMER UND EINEM BEHÄLTER
PROCÉDÉ POUR LE TRANSPORT D'UN MATÉRIAU EN VRAC PARTICULAIRE STÉRILE ET SON TRANSFERT DANS UN ISOLATEUR, AINSI QU'UN CONTENANT, UN ISOLATEUR ET UNE COMBINAISON D'UN ISOLATEUR ET D'UN CONTENANT

(30) Priority: 29.01.2016 DE 102016001027
(43) Date of publication of application: 05.12.2018
(73) Proprietor: Atec Pharmatechnik Gmbh, 24966 Sörup (DE)
(72) Inventor: MUMM, Hans-Werner, 24966 Sörup (DE)
(74) Representative: Weidner Stern Jeschke
(86) International application number: PCT/EP2017/000086
(87) International publication number: WO 2017/129362

(56) References cited:
- EP-A1- 1 510 227
- EP-A1- 1 593 622
- EP-A1- 2 502 636
- EP-A1- 2 823 828
- EP-A1- 3 117 840
- WO-A1-00/74735
- WO-A1-2007/147538
- WO-A1-2009/111019
- WO-A2-2016/202716
- DE-U1- 202004 006 896
- DE-U1- 9 421 818
- US-A1- 2004 005 259

## Description

The invention relates to a process for the transport of a sterile particulate bulk material, particularly of cleaned and sterilized small objects or goods, such as stoppers for infusion bottles and vials, within a container and for the transfer of the particulate bulk material from the container into an isolator according to the preamble of claim 1.

In the pharmaceutical industry, stoppers made of rubber or plastics are required for closing vials and infusion bottles. These stoppers must be washed, sterilized and dried before they can be supplied to a machine for sealing the vials or infusion bottles. The cleaning and sterilization of the stoppers or of similar small objects or goods, such as container lids, parts of syringes or the like, can be carried out in a treatment device as described, for example, in EP 2 823 828 A1. These treatment devices are adapted to receive a treatment and/or transport container into which the small objects are filled through a filling and emptying opening for treatment within the container by means of water, hot steam, drying air and possibly other treatment media. The small objects can either be transferred directly from the container received by the treatment device into an isolator by rotating the container to such an extent that the filling and emptying opening faces downward, as described, for example, in EP 2 823 828 A1. Otherwise, the container is removed from the treatment device and transported to the isolator where it is lifted and turned in order to transfer the material into the isolator as described, for example, in EP 1 769 889 B1.

Similar containers can also be used when the material is only washed in the treatment device or when pre-cleaned material is filled into the container and when the container with the material is moved through an autoclave during the transport to the isolator in order to sterilize the material within the container.

EP 1 510 227 A1 discloses a container for transporting a sterile particulate bulk material, in particular cleaned and sterilized small objects, such as stoppers for infusion bottles and vials, and for transferring the material from the container into an isolator, the container having a filling and emptying opening and a beta part of a RTP system that is attached to the filling and emptying opening and comprises means for docking to a complementary alpha part of the RTP system. Like the container in EP 2 823 828 A1 or a container disclosed in DE 20 2004 006 869 U1 a sieve or mesh is fixedly mounted within the container so that in an upright position of the container liquid treatment media can drop off from the small objects. EP 3 117 840 A1 discloses a container for transporting a sterile particulate bulk material and having a split butterfly valve at the filling and emptying opening.

WO 2007/147538 A1 discloses a liquid disinfection apparatus for cleaning of objects, such as health care objects, within a chamber. The apparatus encloses a wash cart in the form of a basket rack with multiple levels that is guided by rails through a door into the chamber. An inlet port and an outlet port of the apparatus are of a rapid transfer port type.

EP 1 593 622 A1 discloses a transport container for sterile product having a rapid transfer port for docking it to an succeeding apparatus.

In order to enable a transfer of the sterile material from the sterile interior of the container into the sterile interior of the isolator without any contact to a non-sterile environment, a so-called RTP (Rapid-Transfer-Port) system is used. The RTP system comprises one part that is fixedly attached to the filling and emptying opening of the container and is usually referred to as beta part, and a complementary part that is fixedly mounted in a wall of the isolator and is usually referred to as alpha part. Before transferring the material, the container docked to the isolator by mechanically coupling the beta part to the alpha part.

The alpha part, i.e. the part on the side of the isolator comprises a port hole that is surrounded by an annular flange with a sealing gasket, as well as a port door that tightly closes the port hole and can only be opened when the container is docked or coupled to the isolator. The beta part, i.e. the part on the side of the container, comprises a complementary annular flange that includes a sealing gasket and is arranged in front of the filling and emptying opening of the container, as well as a container lid that tightly seals the filling and emptying opening during the transport of the container. When the container is docked to the isolator, on the one hand the two annular flanges of the beta part and the alpha part are mechanically coupled with each other and, on the other hand the container lid and the port door are mechanically coupled with each other. The coupling can be done, for example, by rotating the container about the central axis of the port hole with respect to the isolator. After the container lid has been coupled to the port door, opposing non-sterile outer surfaces of the container lid and of the port door are tightly pressed against each another or are sealingly enclosed between the port door and the container lid. Subsequently, in the coupled state, the port door and the container lid are moved together into an opening position within the isolator in order to open the filling and emptying opening and the port hole, for the transfer of the sterile material. After having transferred the material, the port door and the container lid are moved back into a closed position in order to close the filling and emptying opening and the port hole respectively, then unlock or uncouple the two annular flanges as well as the container lid and the port door, and finally dock off the container from the isolator by uncoupling and disconnecting the two complementary parts of the RTP-system and then move it away.

When the material is transferred into the isolator, any contamination of the isolator must be prevented. When, after the docking, the two annular flanges as well as the port door and the container lid are coupled to each other, their opposing non-sterile surfaces abut against each other and cover each other almost completely, however with the exception of a narrow annular surface at an inner edge of the two opposing non-sterile surfaces of the annular flanges as well as a narrow annular surface at an outer edge of the two opposing non-sterile surfaces of the port door and the container lid. These annular surfaces are also called "rings of concern" because they are a cause of concern with regard to sterility. When the material falls into the isolator after the opening of the port door and the container lid, some of the small objects may get into contact with the "ring of concern" on the inner edge of the opposing non-sterile surfaces of the annular flanges. In this case, there is a risk that microbial contaminants or endotoxins will be transferred from this "ring of concern" to these objects and then into the isolator where they will cause a contamination.

In order to avoid this risk, frequently a movable chute and a glove that is accessible from outside are provided within the isolator. After introducing a hand of an operator into the glove the chute can be grasped and moved in front of the port hole in order to guide the material past or beyond the "ring of concern" without any contact to the latter. The chute also serves to guide the material to a desired position within the isolator. However, such a chute will cause additional costs. Furthermore, it can only be moved in front of the port hole after the port door and the container lid have been opened since otherwise it would block the path of the port door and the container lid. Therefore, any transfer of material into the isolator must be prevented before the chute has been moved in front of the port hole. This is usually done by means of a flap valve of the container that closes the filling and emptying opening and that is opened only after the positioning of the chute in front of the port hole. However, such a flap valve is mostly not present in small containers, whereas in the case of larger containers, it undesirably increases their overall height and thus the space required at the RTP for turning over the container.

Accordingly it is the aim of the invention to improve a process, a container and a combination of a container and an isolator of the type mentioned in the beginning in such a way that the material within the container can be safely transferred past the "ring of concern" and without any contact to the latter to a desired position within the isolator without having to provide the isolator with a movable chute and without having to provide the container with a flap valve.

In order to achieve this object, the process according to the invention is characterized in that the material is transported within the container in a basket, and that after the opening of the RTP, i.e. the port door and the container lid, and before the transfer of the material into the isolator the basket is moved out of the container through the filling and emptying opening and the open RTP into an inclined position inside the isolator in which position the material can slide or fall by itself downwardly out of the basket.

The container according to the invention is characterized in that it comprises a basket for receiving the material to be transported, that the basket, when the RTP is open, is at least partially moveable through the filling and emptying opening and the open RTP out of the container into an inclined position inside the isolator, in which position the material can slide or fall by itself downwardly out of the basket, in that both the container and the basket have a generally cylindrical shape, in that the filling and emptying opening and the beta part of the RTP system are arranged at one end of the container, and in that the basket has an inlet and outlet opening that is adjacent to said one end of the container and faces downwardly in the inclined position of the basket.

The idea of the invention is to use the basket as a replacement for the chute, however to transport this replacement within the container and to withdraw it from the container after the RTP has been opened in order to bring it into the inclined position within the isolator. In the inclined position, the basket will act as a bridge that is located above the "ring of concern" on the inner edge of the two opposing non-sterile surfaces of the annular flanges such that the objects that slide or fall out of the basket will pass the "ring of concern" within the basket. Thus any contact of the objects with the "ring of concern" is prevented. In such a way a chute within the isolator can be dispensed with.

Further the use of a basket inside the container facilitates the cleaning and sterilization of the objects within the container. For this purpose, the walls of the basket are expediently made of perforated sheet metal or of wire mesh, so that during the treatment of the material in the container the treatment media can be passed through the basket and the material within the basket.

Advantageously, both the container and the basket have a generally cylindrical shape. The filling and emptying opening of the container and the beta part of the RTP system and an inlet and outlet opening of the basket are located at the same end of the container such that in the inclined position of the basket the inlet and outlet opening is directed downwards.

In order to make it easier for the operator to move the basket out of the container and into the inclined position by means of a glove protruding into the isolator, the basket is preferably provided with a handle. The handle is pivotal with respect to an annular rim that surrounds the inlet and outlet opening of the basket. In one end position the handle rests against an outer circumference of the rim so that its space requirement in the container is low.

In order to be able to guide the material in the basket to a desired location within the isolator, according to a further preferred embodiment of the invention the basket is moved into an inclined position having a defined inclination angle and is maintained in this position, preferably on the support that is arranged below the alpha part of the RTP system in the interior of the isolator. The support will hold the basket in the desired angle of inclination, preferably between 30 and 70 degrees, most preferably between 40 and 60 degrees with respect to the horizontal.

According to a further preferred embodiment of the invention, the basket is moved out of the container only so far that its rear end still projects into the container. As an alternative or in addition to the support, the basket and/or the container are provided with retaining means, preferably cooperating retaining means that may be also used to retain the basket in the inclined position and, if appropriate, provide the desired angle of inclination. As a result, it is also possible to reliably prevent the basket from dropping or tilting down from the support.

The basket advantageously has a perforated cover which can be pivoted about a pivot axis that is perpendicular to a longitudinal center axis of the basket and which can be locked in the closed state. In this way a flap valve at the container can be dispensed with. In addition it can be ensured, that no material from the basket can slide ore fall into the isolator when the RTP is opened. The pivot axis is advantageously horizontally aligned in the inclined position of the basket in order to ensure a rapid and unobstructed emptying of the basket after pivoting the perforated cover into the open position. Such an orientation of the pivot axis is preferably achieved by moving the basket along a linear guide of the container through the filling and emptying opening and the port hole. In cooperation with a defined rotational alignment of the container in the docked state this provides for the desired orientation.

The pivot axis of the handle is preferably vertical and orthogonal to the pivot axis of the cover so that the handle retains its position when released and cannot pivot downwards by its own weight.

The dimensions of the filling and emptying opening of the container or the dimensions of the opening of the beta part of the RTP system, i.e. the opening that is closed by container lid, are preferably somewhat larger than the maximum cross-sectional dimensions of the basket so that the latter can be removed from the container, if necessary, after having released the retaining means.

In order to prevent the basket from colliding with the open port door or the container lid that is coupled with the port door, or that the support prevents the opening of the port door and the container lid, when the RTP is opened, the port door and the container lid are expediently pivoted about a vertical or steeply inclined pivot axis towards one side, preferably to the side facing away from the glove that protrudes into the isolator.

A further preferred embodiment of the invention provides that the alpha part of the RTP system is integrated in a vertical wall of the isolator. This makes it easier to ensure a laminar air flow along the wall, compared to an integration into a horizontal wall according to EP 1 769 889 B1 or into oblique walls of a projection or recess of the isolator. In addition, due this arrangement the container does not have to be completely or partially turned over in order to empty its content into the isolator. Instead the longitudinal center axis of the container can be horizontal when the container is docked to the isolator, since the basket will be moved out of the container into an inclined position in order to empty it. Thus the vertical space required for the material transfer can be considerably reduced.

In principle, however, it is also possible to integrate the alpha part of the RTP system into an obliquely upward-pointing wall of the isolator. In this case, after docking the container the basket is already in an inclined position, so that after the opening of the RTP, it only has to be pulled out of the container and does not need to be further moved into an inclined position. In this case, a chute within the isolator can be dispensed as well.

If the container according to the invention is used either only for washing or for washing and for sterilizing bulk material in the form of particulate small objects in a treatment device, as described, for example, in EP 2 823 828 A1, the container is preferably provided with two media ports for feeding treatment media, like water, hot steam or drying air into the container and, after passing the material in the basket, for removing the treatment media from the container. One of the media ports may be omitted if the treatment device is provided with a docking, opening and closing device for docking the beta part of the RTP system, as described in EP 2 881 124 A1. In this case treatment media can be fed into or removed from the container through a media port of the docking, opening and closing device and the beta part of the RTP system. Both media ports can be omitted if the container is filled with pre-washed material and the material within the container is sterilized in an autoclave.

### SHORT DESCRIPTION OF THE DRAWINGS

In the following the invention is explained in more detail with reference to the attached drawings. In the drawings
FIG. 1 is an exploded perspective view of a container according to the invention having a basket and beta part of a RTP system;
FIG. 2 is a perspective view of the container after insertion into a mobile carriage;
FIG. 3 is a perspective view of the container after docking it to a treatment device;
FIG. 4 is a partial sectional enlarged front view of the container and a docking, opening and closing device of the treatment device;
FIG. 5 is a sectional view of a portion of a wall of an isolator with a complementary alpha part of the RTP system after the docking of the container;
FIG. 6 is a view like FIG. 5, however after the opening of the RTP;
FIG. 7 is a view like FIG. 6, however after the partial removal of the basket from the container;
FIG. 8 is a view like FIG. 7, however after moving the basket into an inclined position on a support of the isolator;
FIG. 9 is a view like FIG. 8, however after the opening of a cover of the basket;
FIG. 10 is a sectional view of a further container according to the invention that is provided with a filter.

### DETAILED DESCRIPTION OF THE PREFERRED EMBODIMENTS

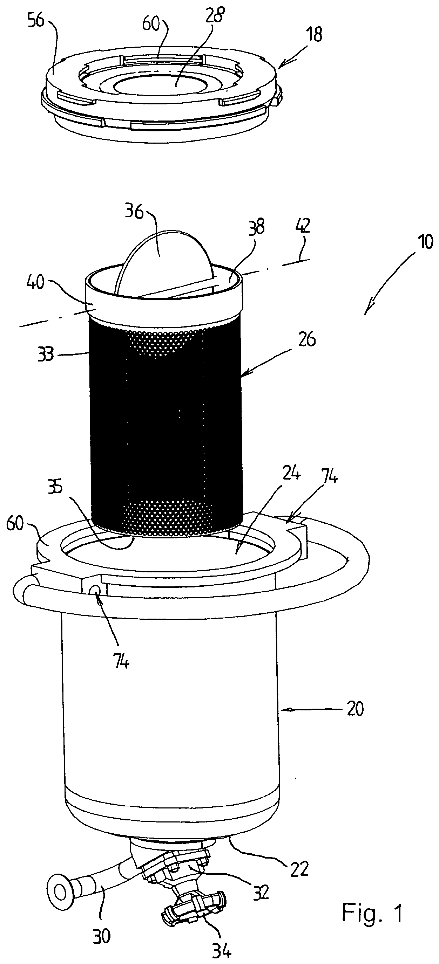

The container 10 as illustrated in FIGS. 1 to 9 is used for the cleaning and sterilization of stoppers made of rubber or plastics that are destined for attachment on vials or infusion bottles (not shown). Cleaning and sterilization are performed by means of a treatment device 12 where the stoppers within the container 10 are treated with various treatment media, such as hot water, hot steam and drying air. Thereafter the container 10 with the cleaned and sterilized stoppers is transported and docked to an isolator 14. Then the stoppers are transferred from the container 10 into the isolator 14, where the stoppers are then applied mechanically to the vials or infusion bottles. The container 10 is intended in particular for users who require smaller amounts of stoppers for this purpose.

In order to prevent any contamination of the sterile material in the container 10 during the transport and the docking to the isolator 14 and during the transfer of the stoppers, the isolator 14 and the container 10 are equipped with a RTP system. The isolator 14 is provided with an alpha part 16 of the RTP system, while the container 10 is provided with a complementary beta part 18 of the RTP system. A suitable RTP system is offered, for example, by Getinge-LaCalhene.

As can be best seen in FIG. 1, the container 10 comprises a generally cylindrical lower body portion 20 that is closed at one end by a bottom 22 and has a filling and emptying opening 24 for the stoppers at the other end. The container 10 includes a basket 26 that is inserted into the lower portion 20. The container 10 further comprises the beta part 18 of the RTP system that is attached to the filling and emptying opening 24 and includes a container lid 28. At the bottom 22 the container 10 is provided with a media port 30 that can be closed by means of a shut-off valve 32 with a hand wheel 34, as best shown in FIG. 1.

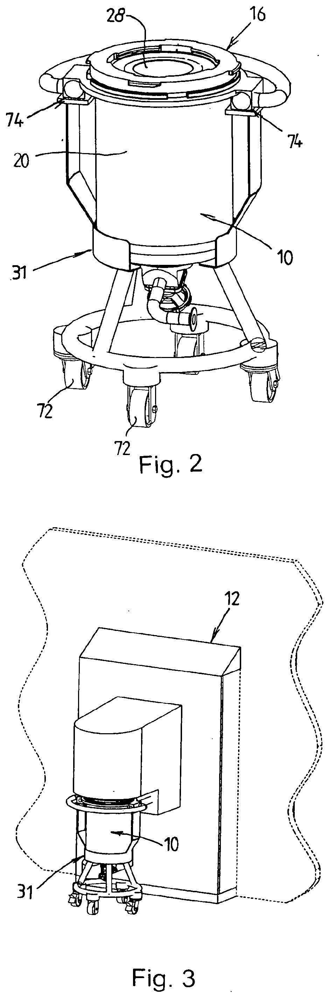

For the treatment the stoppers are filled into the basket 26 of the container 10 while the container lid 28 is open. After the container lid 28 has been closed and the container 10 has been inserted into a mobile carriage 31 (FIG. 2), the container 10 is docked to the treatment device 12 (FIG. 3). After the container lid 28 has been opened the treatment media can flow out of the treatment device 12 through the filling and emptying opening 24 into the container 10, inside the container through the basket 26 and the contents of the basket 26 and then again out of the container 10 through the media port 30 into the treatment device 12, or vice versa.

The generally cylindrical basket 26 has a perforated peripheral wall 33 and a perforated bottom 35 at the lower end in FIG. 1. The bottom 35 may be provided with protruding feet (not shown) so that after the insertion of the basket 26 into the container 10 the bottom 35 is arranged at a distance from the container base 22. At the upper end, the basket 26 is provided with a perforated cover 36, which in the closed state is permeable to the treatment media, but not to the objects in the basket 26. The round cover 36 seals a circular inlet and outlet opening 38 through which the stoppers are introduced into the basket 26 after having opened the cover 36 and through which the stoppers are discharged from the basket 26. The cover 36 is pivotally attached to a rim 40 that surrounds the opening 38 and can be pivoted about a pivot axis 42 that extends diametrically through the opening 38.

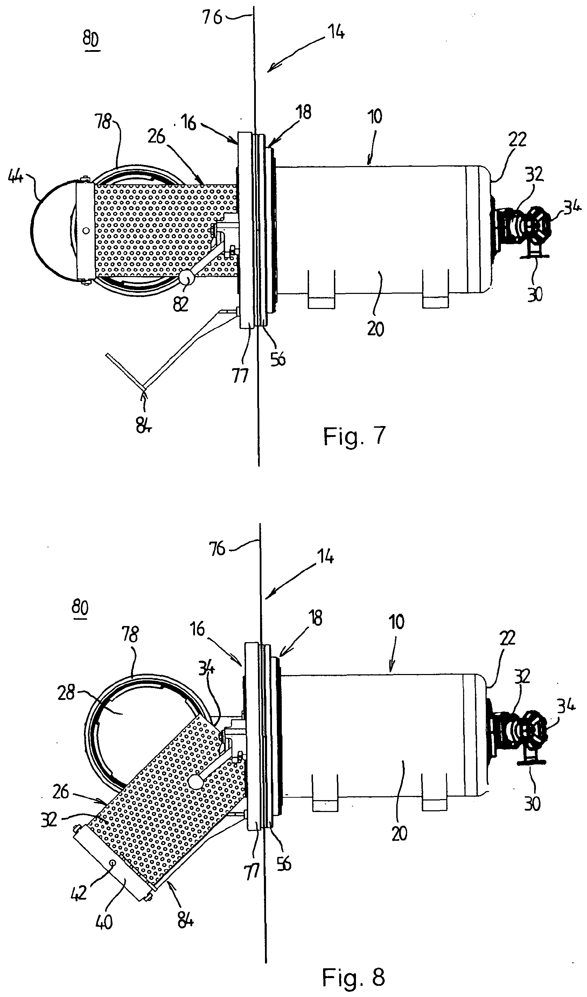

At the upper end, the basket 26 is provided with a handle 44 (FIG. 7) whose ends are pivotally attached to the rim 40 at opposite sides of the opening 38. The pivot axis of the handle 44 is orthogonal to the pivot axis 42 of the cover 36. The semi-circular handle 44 has a slightly larger diameter than the rim 40 so that it can be pivoted to a position where it surrounds one half of the opening 38, rests against the outer periphery of the rim 40 and requires little space inside the container 10.

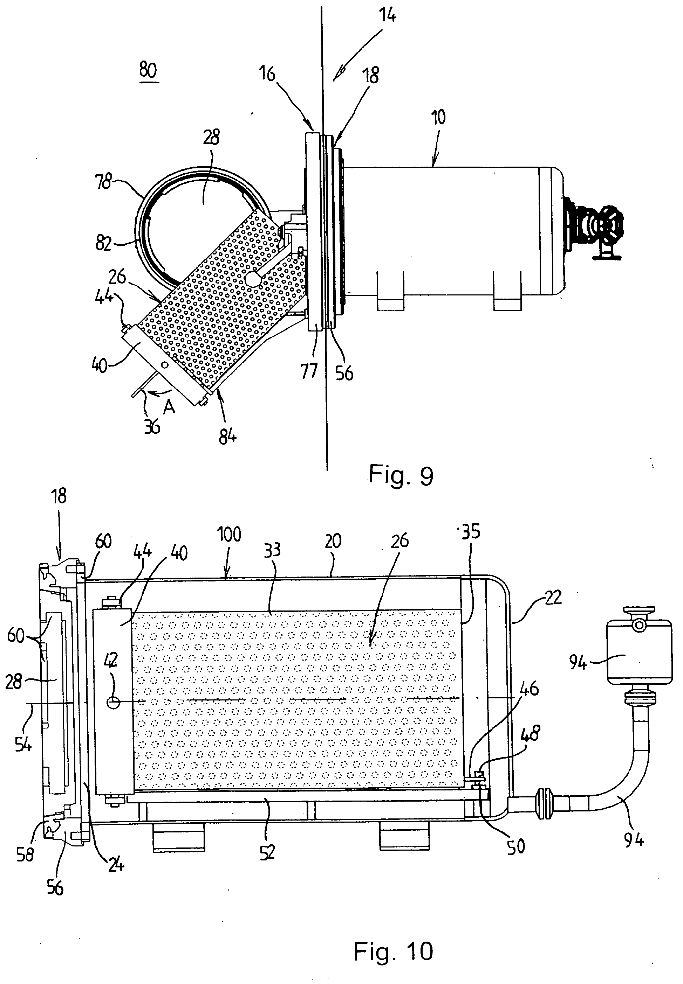

As shown in FIG. 10, the basket 26 is provided with an eyelet 46 which projects beyond the bottom 35 in the vicinity of the peripheral wall 33. In a defined angular orientation of the basket 26 relative to the container 10, the eyelet 46 can be hooked into a protruding hook 48 of a slider 50 that is moveable in parallel to a longitudinal center axis 54 of the container 10 and the basket 26 in a linear guide 52 of the container lower portion 20 into the vicinity of the filling and emptying opening 24. When the eyelet 46 is hooked into the hook 48, the basket 26 in the container 10 can no longer be turned around the longitudinal center axis 54 and can no longer be completely moved out of the container 10.

As also shown in FIG. 10, the beta part 18 of the RTP system on the side of the container 10 comprises an annular flange 56 that surrounds the filling and emptying opening 24, is bolted to an end flange 60 of the container lower portion 20 and comprises an annular sealing gasket 58 inserted in the annular flange 56. The beta part 18 further comprises the removable container lid 28 that has an outer conical surface, which is sealingly pressed against a complementary inner conical surface of the annular gasket 58 in the closed state of the lid 28. On its upper or outer side, the container lid 28 is provided with locking and engagement means 60 that make it possible to open and close the container lid 28 in the treatment device 12 and after the docking to the alpha part 16 of the isolator 14.

For the docking of the container 10, as well as for the opening and closing of the container lid 28, the treatment device 12 comprises a docking, opening and closing device 62. In order to clean and sterilize the material in the container 10 treatment media can be pumped from the treatment device 12 past the open container lid 28 through the docking, opening and closing device 62 into the container 10 or from the container 10 to the treatment device 12. As they pass the conical surfaces of the annular seal 58 and of the open container lid 28 these surfaces are cleaned and sterilized as well.

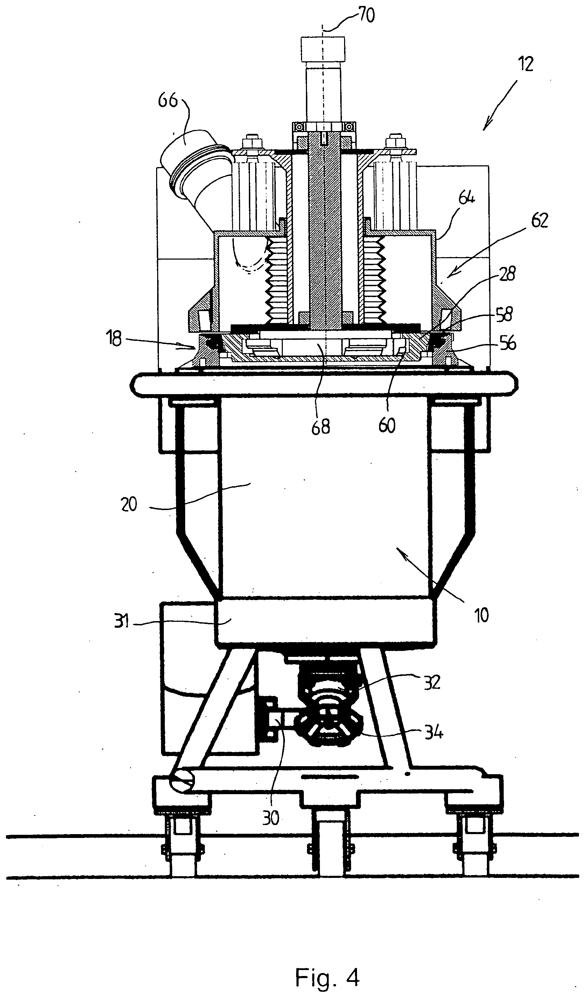

As best shown in FIG. 4, the docking, opening and closing device 62 comprises a downwardly open but otherwise closed cylindrical hood 64 which can be lowered onto the annular flange 56 of the beta part 16 and pressed sealingly against it. A media port 66 connected to the treatment device 12 opens into the hood 64. Treatment media from the treatment device 12 can be fed through the media port 66 into the hood 64 and from there into the container 10. Alternatively treatment media from the container 10 can be discharged through the hood 64 and the media port 66 into the treatment device 12.

Within the hood 64 there is an engaging tool 68 which is raised and lowered relative to the hood 64 and rotated about its vertical axis of rotation 70 first to engage the locking and engaging means 60 of the container lid 28, thereafter to turn the container lid 28 for unlocking and then to lift it out of the annular flange 56 and the annular seal 58 for opening the container lid 28. Once the container lid 28 is open, treatment media, among others hot steam, can flow past the conical surfaces of the annular seal 58 and of the open container lid 28 into and out of the container 10 so that these conical surfaces can be sterilized by the contact with the hot steam. For closing the filling and emptying opening 24 of the container 10 the engagement tool 68 can be lowered together with the container lid 28 until the lid 28 bears against the annular seal 58 and then can be rotated in the opposite direction to interlock the container lid 28 with the annular flange 56, thereafter to disengage the engagement tool 68 from the container lid 28 and lastly to lift the engagement tool 68 again without the container lid 28.

In order to bring the container 10 into a defined positional relationship with respect to the treatment device 12 during docking, to prevent the container 10 from moving and to retain it in the defined positional relationship during the treatment, the mobile carriage 31 has lockable wheels 72. The container 10 and the docking, opening and closing device 62 have co-operating alignment means 74 which ensure that the longitudinal center axis 54 of the container 10 is aligned with the axis of rotation 70 of the engagement tool 68 and that a downwardly facing end face of the hood 64 is pressed against the opposite upper end face of the annular flange 56 without any gap and in a parallel relationship.

The docking, opening and closing device 62 and the alignment means 74 as well as their function are described in more detail in previously cited EP 2 881 124 A1, the disclosure of which is hereby into the present application.

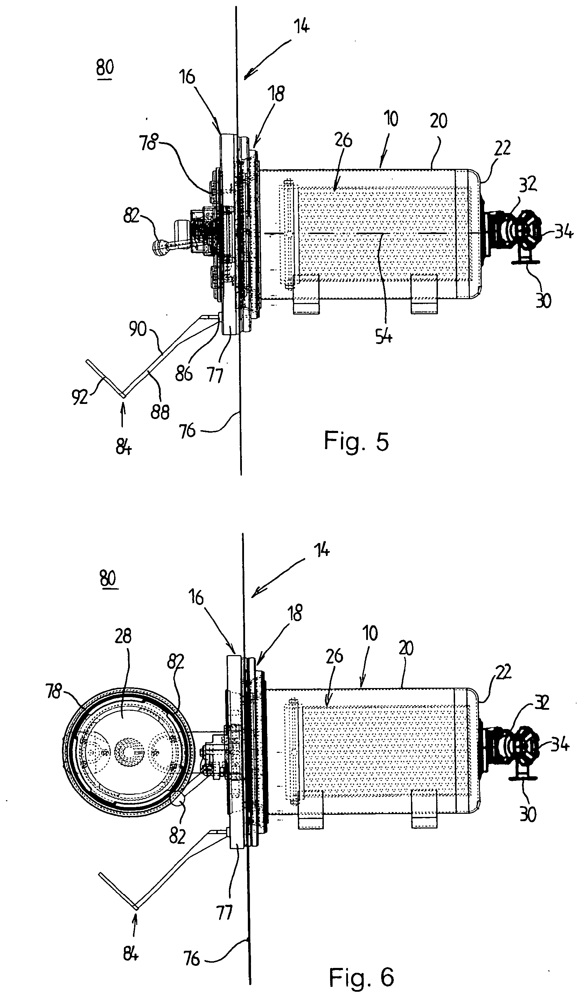

After the treatment of the closure stoppers, the container 10 is unloaded from the treatment device 12 and transported to the isolator 14, where the container 10 is docked to the isolator 14 for the transfer of the cleaned and sterilized stoppers, as best shown in FIG. 5.

As best shown in FIGS. 5 to 9, the alpha part 16 of the RTP system is mounted in a vertical wall 76 of the isolator 14. To dock the beta part 18 of the container 10 to the alpha part 16, the container 10 is rotated about its horizontal longitudinal center axis 54 after the container 10 has been approached to the isolator 14, in order to mechanically couple the parts 16, 18 together.

As known per se the alpha part 16 comprises an annular flange 77 that is rigidly and sealingly mounted in a wall opening of the wall 76, and an annular sealing gasket (not visible) that is inserted in the flange 77. The flange 77 and the gasket are annular and surround a circular port hole (not visible). The alpha part 16 further comprises a pivotal port door 78 having a circular contour that is mounted on the annular flange 77 and normally seals the port hole. After the docking of the container 10 to the alpha part 16 the port door 78 can be opened together with the container lid 28 in order to open the RTP and the filling and emptying opening 24. To this end, near the port hole the isolator 14 is provided with a glove (not shown) that projects through the wall 76 into the interior 80 of the isolator 14. In order to actuate and unlock an unlocking lever 82 of the port door 78 an operator can insert a hand into the glove and grasp the lever 82, in order to open the port door 78 and the container lid 28, as shown in FIG. 6, by pivoting them together into the interior 80 of the isolator 14, about a vertical pivot axis that is arranged laterally adjacent to the port hole.

In addition, the alpha part 16 includes locking means (not shown) that are complementary to the locking and engagement means 60 of the beta part 18. During the rotation of the container 10 when docking, the locking means interlock such that, on the one hand, the two annular flanges 77 and 56 of the alpha part 16 and of the beta part 18 are rigidly coupled with each other and, on the other hand, the container lid 28 is interlocked with the port door 78. In this way the container 10 is docked to the isolator 14 in a preselected rotational orientation. In addition the opposing exterior non-sterile surfaces of the two annular flanges 77 and 56 as well as of the port door 78 and of the container lid 28 are brought into contact with each other and pressed tightly against one another. The non-sterile surfaces cover each other almost completely, with the exception of two narrow circumferential surfaces that are called "rings of concern". One of these surfaces is designated by the reference numeral 82 in FIGS. 6 and 9 while the other one between the non-sterile surfaces of the annular flanges 77 and 56 is not visible.

Below the alpha part 16 the isolator 14 is provided with a support 84 that projects from the wall 76 into the interior of the isolator 14. During the transfer of the stoppers from the container 10 into the isolator 14 the support 84 retains the basket 26 in an inclined position in order to guide the stoppers to a desired location within the isolator 14 and to prevent the stoppers from contacting the "ring of concern" on the non-sterile surfaces of the annular flanges 77 and 56.

The support 84 consists of a holder 86 that is mounted to the annular flange 77, a support rail 88 that extends obliquely downward from the holder 86 and has a flat support surface 90 for the peripheral wall 33 of the basket 26, and a support fork 92 having a roughly semi-circular shape for supporting the peripheral wall 33 of the basket 26. The inner diameter of support fork 92 corresponds to the outer diameter of the wall 33. In the illustrated isolator 14 the angle of inclination of the support surface 90 is approximately 45 degrees, however depending on the intended trajectory of the stoppers it can be more or less as long as a complete emptying of the basket 26 is ensured.

After opening the port door 78 and the container lid 28, the operator grasps the handle 44 of the basket 46 with the glove, pivots it out of the container 10 and pulls the basket 26 on the handle 44 out of the container 10 into the interior 80 of the isolator 14 until the slider 50 abuts against the front end of the linear guide 52 (FIG. 10). This position of the basket 26 is shown in FIG. 7.

Thereafter, the operator lowers the basket 26 until the latter rests with its peripheral wall 33 on the support 84, as shown in FIG. 8. In this position, the basket 26 still projects slightly through the port hole into the container 10 where the basket 26 is held by the eyelet 46 that is hooked in the hook 48 at the lower edge of the bottom 35. Thus the container 10 can neither slide downwards on the support 84 nor can it tilt downwards over the support fork 92. In this position, the handle 44 is pivoted back by the operator until it rests against the outer surface of the rim 40, as also shown in FIG. 8. Now the basket 26 is held in an inclined position in the interior 80 of the isolator 14, with a defined angle of inclination, where its lower end is disposed at a horizontal distance from the wall 76.

Lastly, the operator opens the perforated cover 36 by pressing the upper half of the cover 36 into the interior of the basket 26. This causes the lower half to move outwardly as indicated by the arrow A in FIG. 9. Then the sterile stoppers slide and/or fall by their own from the basket 26 downwardly into the interior 80 of the isolator 14.

After the basket 26 has been emptied, the operator can move it back into the container 10, close and lock the port door 78 and undock the container 10 from the isolator 14.

In contrast to the container 10 described above, the container 100 shown in FIG. 10 serves to sterilize stoppers that have been washed before. The sterilization occurs before or during the transport of the container 100 to the isolator 14 in an autoclave (not shown) into which the closed container 100 is introduced for this purpose. In the autoclave, the container 100 is first evacuated by means of a line 94 that is connected to the bottom 22 of the container 100 instead of the media port 30, then the container 100 is subjected to hot steam, and finally the condensate is removed by applying a vacuum to the container. In order to prevent the material in the container 100 from being contaminated by an inflow of ambient air when the container 100 is removed from the autoclave, the conduit 92 is provided with a particle filter 94.

## Claims

1. A process for transporting sterile particulate bulk material in a container (10) and for transferring the material from the container (10) into an isolator (14), wherein a beta part (18) of a Rapid Transfer Port system that is attached to a filling and emptying opening (24) of the container (10) is docked to a complementary alpha part (16) of the Rapid Transfer Port system that is mounted to the isolator (14) and wherein after the opening of the Rapid Transfer Port, the material is transferred into the isolator (14), wherein the material is transported in a basket (26) within the container (10), **characterized in that**
after the opening of the Rapid Transfer Port and before the transfer of the material into the isolator (14) the basket (26) is moved out of the container (10) through the filling and emptying opening (24) and the open Rapid Transfer Port into an inclined position inside the isolator (14) in which position the material can slide or fall by its own downwardly out of the basket (24).

2. The process according to claim 1, **characterized in that** the basket (26) is moved along a linear guide (52) of the container (10) through the filling and emptying opening (24) and the open Rapid Transfer Port.

3. The process according to claim 1 or 2, **characterized in that** the basket (26) is moved into an inclined position having a defined angle of inclination and is retained in this position.

4. The process according to one of the preceding claims, **characterized in that** the basket (26), in the inclined position, is retained by a support (84) that is disposed inside the isolator (14) below the alpha part (16) of the Rapid Transfer Port system (16, 18).

5. The process according to one of the preceding claims, **characterized in that** the basket (26) is retained in the inclined position by retaining means (46, 48; 84, 92) inside the container (10) and/or inside the isolator (14).

6. The process according to one of the preceding claims, **characterized in that** a perforated cover (36) of the basket (26) is opened in the inclined position of the basket (26).

7. The process according to claim 6, **characterized in that** one half of the cover (36) is pivoted out of the basket (26) about a pivot axis (42).

8. A container (10) for transporting a sterile particulate bulk material and for transferring the material from the container (10) into an isolator (14), the container (10) having a filling and emptying opening (24) and a beta part (18) of a Rapid Transfer Port system (16, 18) that is attached to the filling and emptying opening (24) and comprises means (60) for docking to a complementary alpha part (16) of the Rapid Transfer Port system (16, 18), wherein the container (10) comprises a basket (26) for receiving the material to be transported, whereas both the container (10) and the basket (26) have a generally cylindrical shape, **characterized in that** the filling and emptying opening (24) and the beta part (18) of the Rapid Transfer Port system (16, 18) are arranged at one end of the container (10), **characterized in that** the basket (26), when the Rapid Transfer Port is open, is at least partially moveable through the filling and emptying opening (24) and the open Rapid Transfer Port out of the container (10) and into an inclined position inside the isolator (14) in which position the material can slide or fall by its own downwardly out of the basket (26), and **in that** the basket (26) has an inlet and outlet opening (38) that is adjacent to said one end of the container (10) and faces downwardly in the inclined position of the basket (26).

9. Container according to claim 8, **characterized by** eyelet and hook retaining means (46, 48) that are arranged inside the container (10) for retaining the basket (26) in the inclined position.

10. Container according to claim 8, **characterized by** a slider (50) that is moveable in parallel to a longitudinal center axis (54) of the container (10) and the basket (26) in a linear guide (52) that is arranged inside the container (10) for guiding the movement of the basket (26).

11. Container according to claim 8, **characterized in that** the basket (26) has a perforated cover (36) that is pivotal about a pivot axis (42) which is perpendicular to a longitudinal center axis (54) of the basket (26).

12. Container according to claim 8, **characterized in that** the basket (26) has a handle (44).

## Patentansprüche

1. Verfahren zum Transportieren von sterilem partikelförmigem Schüttgut in einem Behälter (10) und zum Überführen des Materials aus dem Behälter (10) in einen Isolator (14), wobei ein an einer Befüll- und Entleerungsöffnung (24) des Behälters (10) angebrachter Beta-Teil (18) eines Rapid-Transfer-Port-Systems an einen komplementären Alpha-Teil (16) des Rapid-Transfer-Port-Systems angedockt wird, der am Isolator (14) montiert ist, und wobei nach dem Öffnen des Rapid-Transfer-Ports das Material in den Isolator (14) überführt wird, wobei das Material in einem Korb (26) im Inneren des Behälters (10) transportiert wird, **dadurch gekennzeichnet, dass** nach dem Öffnen des Rapid-Transfer-Ports und vor dem Überführen des Materials in den Isolator (14) der Korb (26) durch die Befüll- und Entleerungsöffnung (24) und den offenen Rapid-Transfer-Port aus dem Behälter (10) heraus in eine geneigte Stellung im Inneren des Isolators (14) bewegt wird, in welcher Stellung das Material von selbst nach unten aus dem Korb (24) gleiten oder fallen kann.

2. Verfahren nach Anspruch 1, **dadurch gekennzeichnet, dass** der Korb (26) entlang einer Linearführung (52) des Behälters (10) durch die Befüll- und Entleerungsöffnung (24) und den offenen Rapid-Transfer-Port hindurch bewegt wird.

3. Verfahren nach Anspruch 1 oder 2, **dadurch gekennzeichnet, dass** der Korb (26) in eine geneigte Stellung mit einem definierten Neigungswinkel bewegt und in dieser Stellung gehalten wird.

4. Verfahren nach einem der vorhergehenden Ansprüche, **dadurch gekennzeichnet, dass** der Korb (26) in der geneigten Stellung durch eine Stütze (84) gehalten wird, die im Inneren des Isolators (14) unterhalb des Alpha-Teils (16) des Rapid-Transfer-Port-Systems (16, 18) angeordnet ist.

5. Verfahren nach einem der vorhergehenden Ansprüche, **dadurch gekennzeichnet, dass** der Korb (26) in der geneigten Stellung durch Haltemittel (46, 48; 84, 92) im Inneren des Behälters (10) und/oder im Inneren des Isolators (14) gehalten wird.

6. Verfahren nach einem der vorhergehenden Ansprüche, **dadurch gekennzeichnet, dass** ein perforierter Deckel (36) des Korbs (26) in der geneigten Stellung des Korbs (26) geöffnet wird.

7. Verfahren nach Anspruch 6, **dadurch gekennzeichnet, dass** eine Hälfte des Deckels (36) um eine Schwenkachse (42) aus dem Korb (26) herausgeschwenkt wird.

8. Behälter (10) zum Transportieren eines sterilen partikelförmigen Schüttguts und zum Überführen des Materials aus dem Behälter (10) in einen Isolator (14), wobei der Behälter (10) eine Befüll- und Entleerungsöffnung (24) und einen Beta-Teil (18) eines Rapid-Transfer-Port-Systems (16, 18) aufweist, der an der Befüll- und Entleerungsöffnung (24) angebracht ist und Mittel (60) zum Andocken an einen komplementären Alpha-Teil (16) des Rapid-Transfer-Port-Systems (16, 18) umfasst, wobei der Behälter (10) einen Korb (26) zur Aufnahme des zu transportierenden Materials umfasst, wobei sowohl der Behälter (10) als auch der Korb (26) eine im Allgemeinen zylindrische Form aufweisen, **dadurch gekennzeichnet, dass** die Befüll- und Entleerungsöffnung (24) und der Beta-Teil (18) des Rapid-Transfer-Port-Systems (16, 18) an einem Ende des Behälters (10) angeordnet sind, **dadurch gekennzeichnet, dass** der Korb (26) bei geöffnetem Rapid-Transfer-Port zumindest teilweise durch die Befüll- und Entleerungsöffnung (24) und den offenen Rapid-Transfer-Port hindurch aus dem Behälter (10) heraus und in eine geneigte Stellung im Inneren des Isolators (14) bewegbar ist, in welcher Stellung das Material von selbst nach unten aus dem Korb (26) gleiten oder fallen kann, und dass der Korb (26) eine Einlass- und Auslassöffnung (38) aufweist, die dem genannten einen Ende des Behälters (10) benachbart ist und in der geneigten Stellung des Korbs (26) nach unten weist.

9. Behälter nach Anspruch 8, **gekennzeichnet durch** Ösen- und Haken-Haltemittel (46, 48), die im Inneren des Behälters (10) zum Halten des Korbs (26) in der geneigten Stellung angeordnet sind.

10. Behälter nach Anspruch 8, **gekennzeichnet durch** einen Schieber (50), der parallel zu einer Längsmittelachse (54) des Behälters (10) und des Korbs (26) in einer Linearführung (52) bewegbar ist, die im Inneren des Behälters (10) zum Führen der Bewegung des Korbs (26) angeordnet ist.

11. Behälter nach Anspruch 8, **dadurch gekennzeichnet, dass** der Korb (26) einen perforierten Deckel (36) aufweist, der um eine Schwenkachse (42) schwenkbar ist, die senkrecht zu einer Längsmittelachse (54) des Korbs (26) verläuft.

12. Behälter nach Anspruch 8, **dadurch gekennzeichnet, dass** der Korb (26) einen Griff (44) aufweist.

## Revendications

1. Procédé pour transporter un matériau en vrac particulaire stérile dans un récipient (10) et pour transférer le matériau du récipient (10) dans un isolateur (14), dans lequel une partie bêta (18) d'un système Rapid Transfer Port, qui est fixée à une ouverture de remplissage et de vidange (24) du récipient (10), est accouplée à une partie alpha complémentaire (16) du système Rapid Transfer Port, qui est montée sur l'isolateur (14), et dans lequel, après l'ouverture du Rapid Transfer Port, le matériau est transféré dans l'isolateur (14), dans lequel le matériau est transporté dans un panier (26) à l'intérieur du récipient (10), **caractérisé en ce que**, après l'ouverture du Rapid Transfer Port et avant le transfert du matériau dans l'isolateur (14), le panier (26) est déplacé hors du récipient (10), à travers l'ouverture de remplissage et de vidange (24) et le Rapid Transfer Port ouvert, jusque dans une position inclinée à l'intérieur de l'isolateur (14), position dans laquelle le matériau peut, de lui-même, glisser ou tomber vers le bas hors du panier (24).

2. Procédé selon la revendication 1, **caractérisé en ce que** le panier (26) est déplacé le long d'un guidage linéaire (52) du récipient (10), à travers l'ouverture de remplissage et de vidange (24) et le Rapid Transfer Port ouvert.

3. Procédé selon la revendication 1 ou 2, **caractérisé en ce que** le panier (26) est déplacé dans une position inclinée présentant un angle d'inclinaison défini et est maintenu dans cette position.

4. Procédé selon l'une des revendications précédentes, **caractérisé en ce que** le panier (26), dans la position inclinée, est maintenu par un support (84) qui est disposé à l'intérieur de l'isolateur (14), au-dessous de la partie alpha (16) du système Rapid Transfer Port (16, 18).

5. Procédé selon l'une des revendications précédentes, **caractérisé en ce que** le panier (26) est maintenu dans la position inclinée par des moyens de retenue (46, 48; 84, 92) à l'intérieur du récipient (10) et/ou à l'intérieur de l'isolateur (14).

6. Procédé selon l'une des revendications précédentes, **caractérisé en ce qu'**un couvercle perforé (36) du panier (26) est ouvert dans la position inclinée du panier (26).

7. Procédé selon la revendication 6, **caractérisé en ce qu'**une moitié du couvercle (36) est pivotée hors du panier (26) autour d'un axe de pivotement (42).

8. Récipient (10) pour transporter un matériau en vrac particulaire stérile et pour transférer le matériau du récipient (10) dans un isolateur (14), le récipient (10) ayant une ouverture de remplissage et de vidange (24) et une partie bêta (18) d'un système Rapid Transfer Port (16, 18), qui est fixée à l'ouverture de remplissage et de vidange (24) et comprend des moyens (60) d'accouplement à une partie alpha complémentaire (16) du système Rapid Transfer Port (16, 18), dans lequel le récipient (10) comprend un panier (26) destiné à recevoir le matériau à transporter, tandis que le récipient (10) et le panier (26) présentent tous deux une forme généralement cylindrique, **caractérisé en ce que** l'ouverture de remplissage et de vidange (24) et la partie bêta (18) du système Rapid Transfer Port (16, 18) sont disposées à une extrémité du récipient (10), **caractérisé en ce que** le panier (26), lorsque le Rapid Transfer Port est ouvert, est déplaçable au moins partiellement à travers l'ouverture de remplissage et de vidange (24) et le Rapid Transfer Port ouvert, hors du récipient (10) et jusque dans une position inclinée à l'intérieur de l'isolateur (14), position dans laquelle le matériau peut, de lui-même, glisser ou tomber vers le bas hors du panier (26), et **en ce que** le panier (26) présente une ouverture d'entrée et de sortie (38) qui est adjacente à ladite extrémité du récipient (10) et est orientée vers le bas dans la position inclinée du panier (26).

9. Récipient selon la revendication 8, **caractérisé par** des moyens de retenue à œillet et à crochet (46, 48), qui sont disposés à l'intérieur du récipient (10) pour maintenir le panier (26) dans la position inclinée.

10. Récipient selon la revendication 8, **caractérisé par** un coulisseau (50) qui est déplaçable parallèlement à un axe central longitudinal (54) du récipient (10) et du panier (26) dans un guidage linéaire (52) qui est disposé à l'intérieur du récipient (10) pour guider le mouvement du panier (26).

11. Récipient selon la revendication 8, **caractérisé en ce que** le panier (26) présente un couvercle perforé (36) pivotant autour d'un axe de pivotement (42) qui est perpendiculaire à un axe central longitudinal (54) du panier (26).

12. Récipient selon la revendication 8, **caractérisé en ce que** le panier (26) présente une poignée (44).
